# EUROPÄISCHE PATENTANMELDUNG

(11) **EP 1 070 491 A2**
(43) Veröffentlichungstag der Anmeldung: **24.01.2001**
(21) Anmeldenummer: 00113624.1
(22) Anmeldetag: 28.06.2000
(51) Int. Cl.: A61F 5/01

(54) **Spreizvorrichtung, insbesondere für spastisch gelähmte Kinder**

(30) Priorität: 21.07.1999 DE 19934247
(71) Anmelder: Otto Bock Orthopädische Industrie Besitz- und Verwaltungs-Kommanditgesellschaft, 37115 Duderstadt (DE)
(72) Erfinder: Horacek, Gregor, 56235 Ransbach-Baumbach (DE)
(74) Vertreter: Gramm, Werner, Prof. Dipl.-Ing.

(57) **Zusammenfassung**

Die Erfindung betrifft eine Spreizvorrichtung zur Befestigung im Schritt zwischen und an den Oberschenkeln eines Patienten, insbesondere eines spastisch gelähmten Kindes. Zur Schaffung einer kleinen und unkomplizierten Spreizgehhilfe sind erfindungsgemäß zwei über eine Verschieb- und Drehverbindung (3) miteinander verbundenen Spreizschienen (1,2), die - bezogen auf eine der Medianebene des Patienten entsprechenden Drehebene (15a) - jeweils parallel zu dieser Drehebene (15a) in der Verschieb- und Drehverbindung (3) gegeneinander translatorisch verschiebbar geführt und gleichzeitig um eine senkrecht auf der genannten Drehebene (15a) stehende Drehgelenkachse (4) gegeneinander verdrehbar sind, und die zur Adaptierung einer Orthesenschale oder einer Polsterführung (12) jeweils einen Adapter (10) mit einer Anschlussfläche (10a) aufweisen, wobei die Anschlussflächen (10a) gegenüber der genannten Drehebene (15a) leicht gegeneinander geneigt sind und zwischen sich einen Spreizwinkel (α) einschließen.

## Beschreibung

Die Erfindung betrifft eine Spreizvorrichtung zur Befestigung im Schritt zwischen und an den Oberschenkeln eines Patienten, insbesondere eines spastisch gelähmten Kindes.

Bei vielen spastisch gelähmten Kindern besteht die Tendenz, dass die Hüftgelenke beim Gehen nach innen rotieren und adduzieren - bis hin zur Überkreuzung der Knie. Der Tendenz der Innenrotation und Adduktion begegnet man einerseits mit Nachtlagerungsschienen, die die Beine in einer gespreizten Stellung halten. Der Nachteil dieser Schienen besteht darin, dass sie die Beine starr verbinden, und somit die Bewegungsfreiheit während des Schlafs eingeschränkt wird. Andererseits werden fürs Gehen aufwendige, relativ große und kompliziert anzulegende Gehapparate eingesetzt.

In der US-PS 5,147,286 ist eine Spreizvorrichtung offenbart, die zwei jeweils aus einem Stahldraht gebogene Spreizschienen umfasst, die in Draufsicht angenähert U-förmig ausgebildet und mit ihrem einen U-Schenkel über eine Schraube verbunden sind, deren Achse bei angelegter Spreizvorrichtung in der Medianebene des Patienten liegt. Der in dem genannten Befestigungsbereich liegende U-Schenkel jeder Spreizschiene schließt mit dem zugeordneten U-Steg einen Winkel von etwa 115° ein, so dass die mit einer Anschlussfläche ausgestatteten U-Stege ausgehend von der genannten Schraubverbindung keilförmig auseinanderlaufen. Die beiden Spreizschienen lassen sich um die genannte Schraubenachse gegeneinander verschwenken und in der gewünschten Schwenkstellung durch einen Steckstift verriegeln. Werden die beiden Spreizschienen aus einer gemeinsamen Ebene heraus um die Schraubenachse gegeneinander verschwenkt, ergeben sich gegenüber der genannten Medianebene leicht gegeneinander geneigte Anschlussflächen, die zwischen sich einen Spreizwinkel einschließen.

Die US-PS 5,558,628 offenbart eine weitere Spreizvorrichtung, die in Draufsicht etwa H-förmig ausgebildet ist, wobei der die beiden Schenkel miteinander verbindende H-Steg längenveränderlich ausgebildet ist. Die beiden H-Schenkel weisen in Draufsicht eine konkav gewölbte Außenfläche auf, die der Schenkelform des Patienten angepasst ist. Jeder H-Schenkel ist in seinem mittleren Bereich an dem zugeordneten Ende des H-Steges verschwenkbar festgelegt, wobei die Verschwenkachse jeweils parallel zu der Medianebene des Patienten liegt, der mit dieser Spreizvorrichtung versorgt wird.

Der Erfindung liegt die Aufgabe zugrunde, eine verbesserte Spreizvorrichtung zu schaffen.

Diese Aufgabe wird erfindungsgemäß mit den Merkmalen des Gegenstandes nach dem Anspruch 1 gelöst.

Dabei ist es aus Stabilitätsgründen zweckmäßig, wenn jede Spreizschiene als übereinander liegendes Schienenpaar ausgebildet ist.

Durch die geneigte Anordnung der Adapter-Anschlussflächen schließen diese immer einen Spreizwinkel ein, der durch entsprechende Auslegung der Adapter individuell anpaßbar bzw. justierbar sein kann. Die Adapter können z. B. mittels einer Schraubverbindung (zur weiteren Adaptierung auf eine Orthesenschale) direkt auf eine Orthese (für den inneren Oberschenkel) oder auf eine universelle Polsterführung für den Oberschenkel montiert werden. Zur Sicherung am Oberschenkel bieten sich z. B. Gurte an.

Die so fertiggestellte Spreizgehhilfe wird im Schritt zwischen den Oberschenkeln und an diese angelegt. Um bei gegebener translatorischer Bewegungsfreiheit der Spreizgehhilfe möglichst weite Schritte zu ermöglichen, sollte die Spreizvorrichtung möglichst weit vom Knie entfernt angelegt werden. Durch die gleichzeitig ermöglichte Translation und Rotation können die Hälften der Spreizgehhilfe der Vorwärtsbewegung der Oberschenkel folgen, verhindern aber gleichzeitig - auf-grund ihrer zwangsläufigen gegenseitigen Beabstandung - die Innenrotation der Hüftgelenke.

Bei dem vorstehend beschriebenen Bewegungsablauf erzeugen die Oberschenkel in der Regel einen hohen Druck auf die beiden Hälften der Spreizgehhilfe und somit in deren jeweiliger Gleitführung. Dies erfordert einerseits eine ausreichend große Stabilität der Konstruktion; andererseits sollte aber die Verschieb- und Drehverbindung möglichst schmal ausgelegt werden, um bei gegebener Gesamtbaubreite der Spreizgehhilfe eine möglichst weite translatorische Bewegung zu ermöglichen. Aus therapeutischer Sicht wären hohe im Drehlager sowie in der Verschiebeführung auftretende Reibungskräfte, die der Bewegung der Oberschenkel entgegenwirken, unerwünscht. Um diese Reibungskräfte soweit wie möglich zu reduzieren, wird erfindungsgemäß vorgeschlagen, daß die Verschieb- und Drehverbindung zwei miteinander über die Drehgelenkachse gegeneinander verdrehbar verbundene Lagerblöcke aufweist. Dabei ist es zweckmäßig, wenn jeder Lagerblock mit zumindest einem Linearkugellager für die translatorische Verschiebung der zugeordneten Spreizschiene ausgestattet ist, wobei die Spreizschienen in Material und Abmessung auf die Linearkugellager abgestimmt erden können. Ferner ist es vorteilhaft, wenn die Drehgelenkachse durch eine Senkschraube gebildet ist, die mit Spielpassung durch den einen Lagerblock hindurchgeführt und mit einer den anderen Lagerblock durchdringenden Schulterbuchse veschraubt ist, gegen deren freies Ende der eine Lagerblock anliegt.

Die erfindungsgemäß gestaltete Spreizvorrichtung führt somit zu einer kleinen, unkomplizierten Spreizgehhilfe, die schnell anzulegen ist, dem Patienten genügend Bewegungsfreiheit läßt und eine individuelle Anpassung der Abduktion zuläßt. Zugleich aber kann die erfindungsgemäße Spreizvorrichtung auch als Nachtlagerungsschierie mit Bewegungsmöglichkeiten für die Beine eingesetzt werden.

In der Zeichnung ist eine als Beispiel dienende Ausführungsform der Erfindung dargestellt. Es zeigen
- **Figur 1**: eine Spreizgehhilfe in Seitenansicht;
- **Figur 2**: die Ausführungsform gemäß Figur 1 in Stirnansicht und zum Teil im Querschnitt;

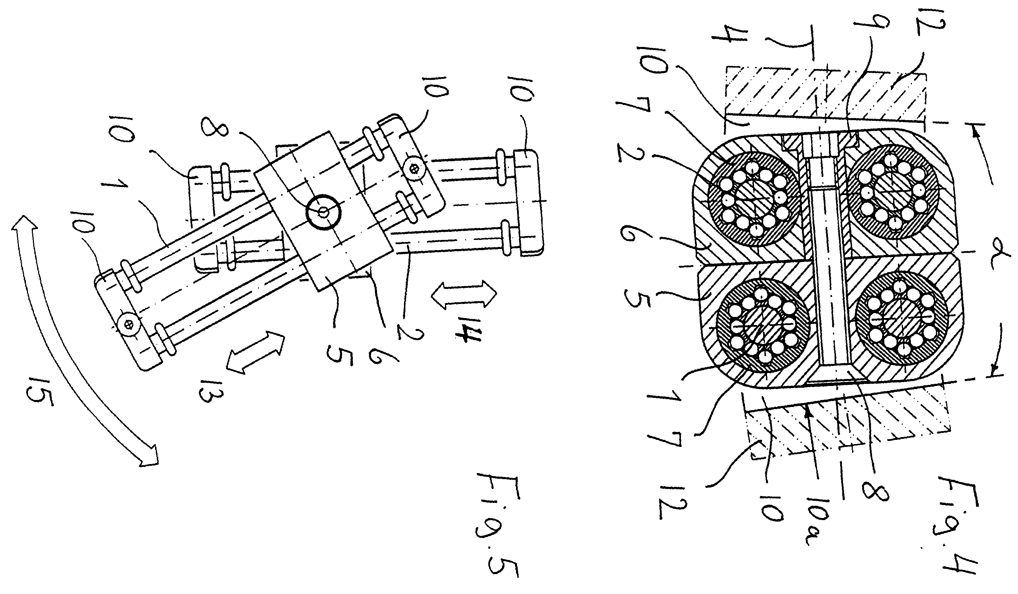

- **Figur 3**: die Ausführungsform gemäß Figur 1 in Draufsicht und zum Teil im Horizontalschnitt;
- **Figur 4**: in vergrößertem Maßstab einen lotrechten Querschnitt gemäß der Linie IV-IV in Figur 1;
- **Figur 5**: in einer Darstellung gemäß Figur 1 die Spreizgehhilfe in verschwenkter Lage;
- **Figur 6**: in einer Darstellung gemäß Figur 2 die mit einer Polsterführung bestückte Spreizgehhilfe;
- **Figur 7**: die Darstellung gemäß Figur 6 in Draufsicht und
- **Figur 8**: in Seitenansicht die Ausführungsform gemäß Figur 6 in drehverschwenkter Lage.

Die Figuren zeigen eine erfindungsgemäß gestaltete Spreizgehhilfe, die im wesentlchen aus zwei Spreizschienenpaaren 1, 2 besteht, die über eine Verschieb- und Drehverbindung 3 gegeneinander translatorisch und rotatorisch miteinander verbunden sind.

Die Verschieb- und Drehverbindung 3 weist zwei miteinander über eine Drehgelenkachse 4 gegeneinander verdrehbar verbundene Lagerblöcke 5, 6 auf, die jeweils mit zwei Linearkugellagern 7 für die translatorische Verschiebung des zugeordneten Spreizschienenpaares 1 bzw. 2 ausgestattet sind (siehe insbesondere Figur 4).

Die Drehgelenkachse 4 ist durch eine Senkschraube 8 gebildet, die mit Spielpassung durch den einen Lagerblock 5 hindurchgeführt und mit einer den anderen Lagerblock 6 durchdringenden Schulterbuchse 9 verschraubt ist, gegen deren freies Ende der eine Lagerblock 5 anliegt. Damit die beiden Lagerblöcke 5, 6 nicht gegeneinander verspannt werden und gegeneinander frei drehbar bleiben, ist die Schaftlänge der Schulterbuchse 9 so gewählt, dass zwischen den beiden Lagerblöcken 5, 6 ein geringer Spalt verbleibt. Der Lagerblock 6 dreht dann auf dem Schaft der Schulterbuchse 9.

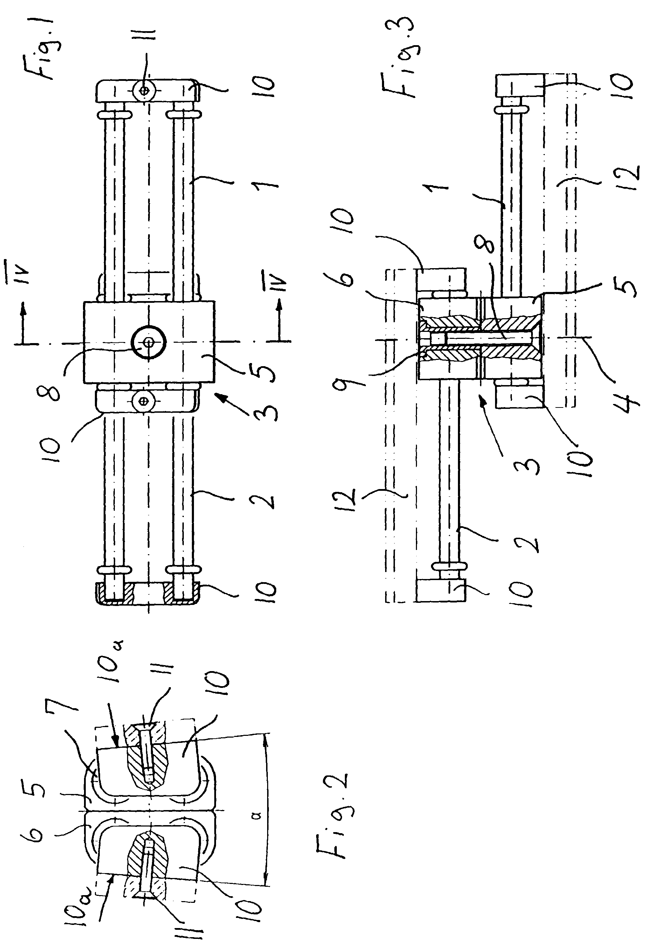

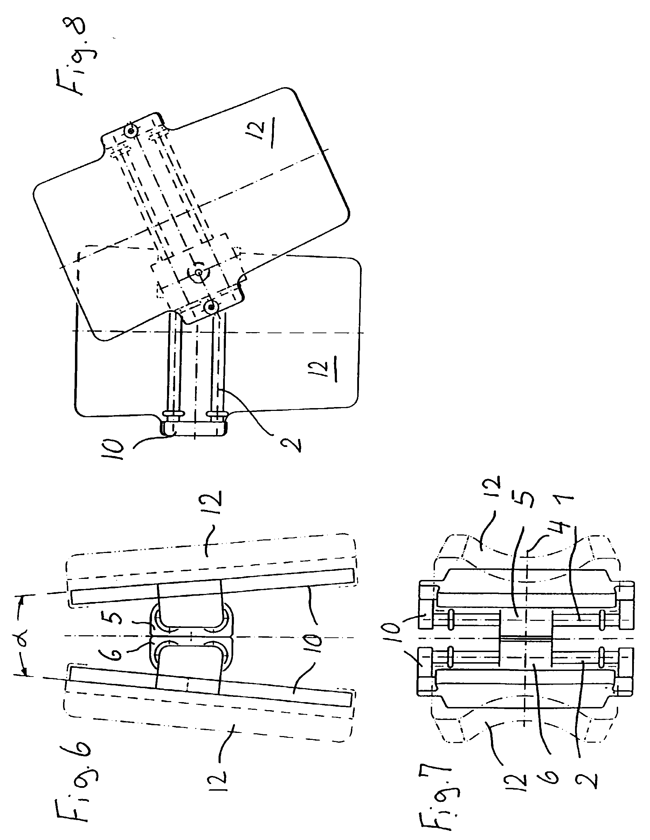

Die Drehgelenkachse 4 steht senkrecht auf einer Drehebene 15a, die der Medianebene des mit der Spreizvorrichtung versorgten Patienten entspricht. Die sich gegenüberliegenden Enden jedes Spreizschienenpaares 1, 2 sind jeweils mit einer Adapterplatte 10 bestückt, deren Anschlussflächen 10a gegenüber der genannten Drehebene 15a leicht gegeneinander geneigt sind und zwischen sich einen Spreizwinkel einschließen (siehe insbesondere Figur 2). Gemäß den Figuren 6 bis 8 kann auf den beiden Adapterplatten 10 jedes Spreizschienenpaares 1, 2 z. B. mittels einer Schraubverbindung 11 eine universelle Polsterführung 12 für den nicht dargestellten Oberschenkel des Patienten montiert werden.

In Figur 5 zeigen die Pfeile 13, 14 die mögliche translatorische Verschiebung jedes Spreizschienenpaares 1, 2 gegenüber dem ihm zugeordneten Lagerblock 5 bzw. 6 und der Pfeil 15 die rotatorische Verschwenkmöglichkeit zwischen den beiden Spreizschienenpaaren 1, 2 um die Drehgelenkachse 4.

## Patentansprüche

1. Spreizvorrichtung zur Befestigung im Schritt zwischen und an den Oberschenkeln eines Patienten, insbesondere eines spastisch gelähmten Kindes, mit zwei über eine Verschieb- und Drehverbindung (3) miteinander verbundenen Spreizschienen (1, 2), die - bezogen auf eine der Medianebene des Patienten entsprechenden Drehebene (15a) - jeweils parallel zu dieser Drehebene (15a) in der Verschieb- und Drehverbindung (3) gegeneinander translatorisch verschiebbar geführt und gleichzeitig um eine senkrecht auf der genannten Drehebene (15a) stehende Drehgelenkachse (4) gegeneinander verdrehbar sind,
und die zur Adaptierung einer Orthesenschale oder einer Polsterführung (12) jeweils einen Adapter (10) mit einer Anschlussfläche (10a) aufweisen, wobei die Anschlussflächen (10a) gegenüber der genannten Drehebene (15a) leicht gegeneinander geneigt sind und zwischen sich einen Spreizwinkel (α) einschließen.

2. Spreizvorrichtung nach Anspruch 1, **dadurch kennzeichnet**, daß jede Spreizschiene (1, 2) als übereinanderliegendes Schienenpaar ausgebildet ist.

3. Spreizvorrichtung nach Anspruch 1 oder 2, **dadurch gekennzeichnet**, daß die Verschieb- und Drehverbindung (3) zwei miteinander über die Drehgelenkachse (4) gegeneinander verdrehbar verbundene Lagerblöcke (5, 6) aufweist.

4. Spreizvorrichtung nach Anspruch 3, **dadurch gekennzeichnet**, daß jeder Lagerblock (5, 6) mit zumindest einem Linearkugellager (7) für die translatorische Verschiebung (13, 14) der zugeordneten Spreizschiene (1, 2) ausgestattet ist.

5. Spreizvorrichtung nach Anspruch 3 oder 4, **dadurch gekennzeichnet**, daß die Drehgelenkachse (4) durch eine Senkschraube (8) gebildet ist, die mit Spielpassung durch den einen Lagerblock (5) hindurchgeführt und mit einer den anderen Lagerblock (6) durchdringenden Schulterbuchse (9) verschraubt ist, gegen deren freies Ende der eine Lagerblock (5) anliegt.

6. Spreizvorrichtung nach einem der vorhergehenden Ansprüche, **dadurch gekennzeichnet**, daß der Spreizwinkel (α) justierbar ist.
